# EUROPÄISCHE PATENTANMELDUNG

(11) **EP 0 770 607 A1**
(43) Veröffentlichungstag der Anmeldung: **02.05.1997**
(21) Anmeldenummer: 96116671.7
(22) Anmeldetag: 17.10.1996
(51) Int. Cl.: C07D 307/20, C11D 1/62, A61K 7/06, A61K 7/48, A61K 7/50

(54) **Sorbitanesterquats und ihre Verwendung als oberflächenaktive Mittel, insbesondere zur Haar- und Körperpflege**

(30) Priorität: 26.10.1995 DE 19539875
(71) Anmelder: Henkel Kommanditgesellschaft auf Aktien, 40589 Düsseldorf-Holthausen (DE)
(72) Erfinder: Bigorra Llosas, Joaquim, Dr., 08203 Sabadell (ES); Subirana, Rafael Pi, Dr., 08400 Granollers (ES); Weuthen, Manfred, Dr., 42697 Solingen (DE)

(57) **Zusammenfassung**

Es werden neue Esterquats auf Zuckerbasis vorgeschlagen, die man erhält, indem man Sorbitanester, gegebenenfalls nach Ethoxylierung, mit Epoxypropylammoniumverbindungen quaterniert. Die Produkte zeichnen sich durch eine gute ökotoxikologische Verträglichkeit und eine ausgezeichnete Avivage gegenüber Textilien und Haaren aus.

## Beschreibung

### Gebiet der Erfindung

Die Erfindung betrifft neue zuckerbasierte Esterquats, die man erhält, indem man Sorbitanester, gegebenenfalls nach Ethoxylierung, mit quaternären Epoxypropylammoniumverbindungen quaterniert.

### Stand der Technik

Unter der Bezeichnung 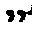 Esterquats" werden im allgemeinen quaternierte Fettsäuretriethanolaminestersalze verstanden, die sich in breitem Umfang sowohl für die Faser- als auch für die Haaravivage eignen. In den vergangenen Jahren haben diese Stoffe infolge ihrer besseren ökotoxikologischen Verträglichkeit konventionelle quartäre Ammoniumverbindungen wie z.B. das bekannte Distearyldimethylammoniumchlorid zu einem guten Teil vom Markt verdrängt. Übersichten zu diesem Thema sind beispielsweise von O.Ponsati in C.R. **CED-Kongress, Barcelona, 1992, S.167 ,** R.Puchta et al. in **Tens.Surf.Det., 30, 186 (1993),** M.Brock in **Tens. Surf. Det. 30, 394 (1993)** und R.Lagerman et al. in **J.Am.Oil.Chem.Soc., 71, 97 (1994)** erschienen.

Kationische Avivagemittel auf Basis von Aminozuckern (Glucamin, Bis-Bissorbitylamin) sind ferner aus den Deutschen Offenlegungsschriften **DE-A1 42 38 207, DE 42 38 212, DE-A1 42 38 213, DE-A1 42 38 216** und **DE-A1 42 38 217** (Henkel) bekannt.

Obschon Esterquats des Stands der Technik über sehr gute anwendungstechnische Eigenschalten verfügen sowie eine zufriedenstellende biologische Abbaubarkeit und eine gute hautkosmetische Verträglichkeit besitzen, gehen die Anforderungen des Verbrauchers weiterhin in die Richtung verbesserter Produkteigenschaften.

Demzufolge hat die Aufgabe der Erfindung darin bestanden, neue Esterquat-Typen zur Verfügung zu stellen, die sich durch eine weiter verbesserte ökotoxikologische Verträglichkeit auszeichnen und gleichzeitig ein gutes Avivage- und Antistatikverhalten aufweisen.

### Beschreibung der Erfindung

Gegenstand der Erfindung sind Sorbitanesterquats, die man erhält, indem man Sorbitanester, gegebenenfalls nach Ethoxylierung, mit Epoxypropylammoniumverbindungen quaterniert.

Es werden neue zuckerbasierte kationische Tenside erhalten, die sich überraschenderweise gegenüber Produkten des Stands der Technik nicht nur durch eine besonders gute ökotoxikologische Verträglichkeit, sondern auch ausgezeichnete Haar- und Faseravivage sowie eine Verminderung der elektrostatischen Aufladung zwischen den Faserfilamenten auszeichnen.

Ein weiterer Gegenstand der Erfindung betrifft ein Verfahren zur Herstellung von Sorbitanesterquats, die man erhält, indem man Sorbitanester, gegebenenfalls nach Ethoxylierung, mit Epoxypropylammoniumverbindungen quaterniert.

### Sorbitanester

Sorbitanester stellen Veresterungs- bzw. Umesterungsprodukte von Sorbitol mit Fettsäuren bzw. Fettsäuremethylestern bei Temperaturen von 200 bis 250°C dar. Dabei wird in einem ersten Schritt Sorbitol zu einer Mischung von 1,4- und 3,6-Sorbitan dehydratisiert [vgl. S.Ropuszynski et al. in **Tens. Surf. Det. 27, 350 (1990)]** und das Sorbitan anschließend - abhängig von den Einsatzbedingungen - in ein Gemisch von Mono-, Di- und Triestern überführt [vgl. C.Akoh et al. in **J. Am. Oil. Chem. Soc., 66, 1581 (1989)** und **Surfactants in Consumer Products, J.Falbe (ed), Springer-Verlag, Berlin, 1987, S.101-103**. Anionisch modifizierte Sorbitanester sind aus der Deutschen Patentanmeldung **DE-A1 39 41 061** bekannt.

Sorbitanester, die im Sinne der Erfindung eingesetzt werden können, folgen der Formel **(I)**, 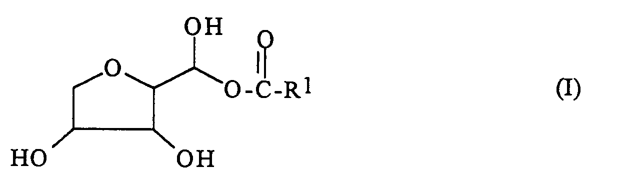 in der R¹CO für einen aliphatischen Acylrest mit 6 bis 22 Kohlenstoffatomen und 0 und/oder 1, 2 oder 3 Doppelbindungen steht. Formel **(I)** gibt exemplarisch einen Monoester wieder. Es versteht sich, daß auch die weiteren Hydroxylgruppen verestert sein können.

Typische Beispiele für geeignete Sorbitanester, vorzugsweise Sorbitanmono-, Sorbitan-sesqui- und/oder Sorbitandiester, sind formale Veresterungsprodukte des Sorbitans mit Ca-pronsäure, Caprylsäure, 2-Ethylhexansäure, Caprinsäure, Laurinsäure, Isotridecansäure, Myri-stinsäure, Palmitinsäure Palmoleinsäure, Stearinsäure, Isosterinsäure, Olsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen.

Bevorzugt sind technische Sorbitanmono-, -sesqui- oder -diester, deren Fettsäurekomponenten sich von einem technischen Kokosfettsäureschnitt mit 12 bis 14 bzw. 12 bis 18 Kohlenstoffatomen, Laurinsäure, Palmitinsäure, Stearinsäure oder Ölsäure ableiten.

### Epoxypropylammoniumverbindungen

Epoxypropylammoniumverbindungen, die im Sinne der Erfindung eingesetzt werden können, folgen beispielsweise der Formel **(II)**, 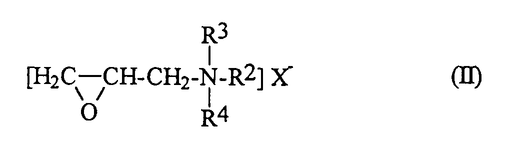 in der R², R³ und R⁴ unabhängig voneinander für gegebenenfalls hydroxysubstituierte Alkylreste mit 1 bis 4 Kohlenstoffatomen und X für Halogen steht. Typische Beispiele sind die Tetramethylammoniumchloride und -bromide, die beispielsweise unter der Marke Quab® von der Degussa AG erhältlich sind.

### Ethoxylierung

Zur Herstellung von polyethylenoxidhaltigen Sorbitanesterquats kann man nach zwei Alternativen verfahren. Zum einen kann man ethoxyliertes Sorbitan einsetzen. Dies hat den Vorteil, daß die Alkylenoxidverteilung im später resultierenden Sorbitanesterquat bezüglich der vier OH-Gruppen des Polyols annähernd gleich ist. Nachteilig ist jedoch, daß die Ver-esterung aus sterischen Gründen schwieriger wird. Die Methode der Wahl besteht daher darin, den Ester vor der Quaternierung zu ethoxylieren. Dies kann in an sich bekannter Weise geschehen, d.h. in Anwesenheit basischer Katalysatoren und bei erhöhten Temperaturen. Als Katalysatoren kommen beispielsweise Alkali- und Erdalkalihydroxide und -alkoholate, vor-zugsweise Natriumhydroxid und insbesondere Natriummethanolat in Betracht; die Einsatz-menge liegt üblicherweise bei 0,5 bis 5 und vorzugsweise 1 bis 3 Gew.-% - bezogen auf die Einsatzstoffe. Bei Verwendung dieser Katalysatoren werden in erster Linie freie Hydroxyl-gruppen alkoxyliert. Setzt man als Katalysatoren jedoch calcinierte oder mit Fettsäuren hydro-phobierte Hydrotalcite ein, kommt es auch zu einer Insertion des Ethylenoxids in die Ester-bindungen. Diese Methode ist bevorzugt, wenn man eine Ethylenoxidverteilung wünscht, die der bei Einsatz von ethoxyliertem Sorbitan nahe kommt. Die Reaktion wird üblicherweise bei Temperaturen im Bereich von 100 bis 180°C durchgeführt. Wegen des empfindlichen Zuckeranteils ist es von Vorteil, bei möglichst niedrigen Temperaturen beispielsweise im Bereich von 110 bis 140°C zu arbeiten und dafür eine etwas längere Reaktionszeit in Kauf zu nehmen. Durch den Einbau von im Durchschnitt 1 bis 25 Mol Ethylenoxid pro Mol Ester wird die Hydrophilie der Sorbitanesterquats gesteigert, die Löslichkeit verbessert und die Reaktivität gegenüber anionischen Tensiden herabgesetzt.

### Ouaternierung

Die Quaternierung der Sorbitanester bzw. der ethoxylierten Sorbitanester ( 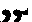 Polysorbate") kann in an sich bekannter Weise durchgeführt werden. Dazu legt man den Ester, gegebe-nenfalls in wäßrigem Isopropylalkohol gelöst bzw. Suspendiert, vor und tropft das Quater-nierungsmittel langsam bei Temperaturen im Bereich von 50 bis 90 und vorzugsweise 65 bis 75°C zu. Es empfiehlt sich, die Mischung auf einen alkalischen pH-Wert im Bereich von 9 bis 10 einzustellen. Nach Beendigung der Zugabe läßt man weitere 24 bis 48 h rühren. Das molare Einsatzverhältnis zwischen Sorbitanester und Quaternierungsmittel hängt von der Zahl der Estergruppen und dem gewünschten Quaternierungsgrad ab. Üblicherweise wird man Sorbitanmonoester mit äquimolaren Mengen des Quaternierungsmittels, also in einem mola-ren Verhältnis von 1 : 0,95 bis 1 : 1,05 einsetzen. Bei Einsatz von Di- oder Triestern kann das molare Einsatzverhältnis zwischen Estergruppe und Quaternierungsmittel ebenfalls 1 : 0,95 bis 1 : 1,05 betragen, wobei natürlich die Möglichkeit besteht, in einen Diester zwei und in einen Triester sogar drei kationische Gruppen einzubauen und auf diese Weise - insbesondere bei Einsatz von ethoxylierten Ausgangsstoffen - Pseudo-Kationpolymere herzustellen.

### Tenside

Die erfindungsgemäßen Sorbitanesterquats können zusammen mit weiteren anionischen, nichtionischen, kationischen und/oder amphoteren Tensiden eingesetzt werden. Wegen des Problems der Adduktbildung zwischen kationischen und anionischen Tensiden sind natürlich Mischungen der Sorbitanesterquats mit nichtionischen, amphoteren und zwitterionischen Tensiden bevorzugt. Sorbitanesterquats, insbesondere solche, die über Polyoxyalky-lengruppen verfügen, besitzen jedoch gegenüber anionischen Tensiden eine vergleichsweise stark herabgesetzte Reaktivität, so daß das Problem der Salzbildung und/oder Inaktivierung in der Praxis kaum zum Tragen kommt.

Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischether-sulfate, Monoglycerid(ether)sulfate, Fettsäureamid(ether)sulfate, Mono- und Dialkylsulfo-succinate, Mono- und Dialkylsulfosuccinamate, Sulfotriglyceride, Amidseifen, Ethercar-bonsäuren und deren Salze, Fettsäureisethionate, Fettsäuresarcosinate, Fettsäuretauride, Acyl-lactylate, Acyltartrate, Acylglutamate, Acylaspartate, Alkyloligoglucosidsulfate, Proteinfett-säurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Typische Beispiele für **nichtionische Tenside** sind Fettalkoholpolyglycolether, Alkylphenolpolyglycolether, Fettsäurepolyglycolester, Fettsäureamidpolyglycolether, Fettaminpolygly-colether, alkoxylierte Triglyceride, Mischether bzw. Mischformale, Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglyeoletherketten enthalten, können diese eine konventionelle, vorzugsweise jedoch eine eingeengte Homologenverteilung aufweisen.

Typische Beispiele für **kationische Tenside** sind quartäre Ammoniumverbindungen und Esterquats, insbesondere quaternierte Fettsäuretrialkanolaminester-Salze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Ber-in, 1987, S. 54-124** oder **J.Falbe (ed.), "Kalysatoren, Tenside und Mineralöladditive", Thieme Verlag, Stuttgart, 1978, S. 123-217** verwiesen. Aus anwendungstechnischer Sicht sind Abmischungen von Sorbitanesterquats mit den genannten Tensiden im Gewichtsverhältnis 10 : 90 bis 90 : 10 bevorzugt. Besonders vorteilhalte Eigenschaften werden bei Kombinationen von Sorbitanesterquats mit Alkyloligoglucosiden, Fettsäure-N-alkylglucamiden und/oder Betainen erhalten.

### Gewerbliche Anwendbarkeit

Die erfindungsgemäßen Sorbitanesterquats weisen ein ausgezeichnetes Avivagevermögen auf, vermindern die elektrostatische Aufladung zwischen synthetischen und natürlichen Fasern, auch Keratinfasern, und zeichnen sich durch besonders vorteilhafte ökotoxikologische Verträglichkeit aus.Ein weiterer Gegenstand der Erfindung betrifft daher ihre Verwendung zur Herstellung von oberflächenaktiven Mitteln wie z.B. Wasch-, Spül-, Reinigungs- und Avivagemitteln sowie vorzugsweise Mitteln zur Haar- und Körperpflege, in denen sie in Mengen von 1 bis 50, vor-zugsweise 3 bis 35 Gew.-% - bezogen auf die Mittel - enthalten sein können.

### Haar- und Körperpflegemittel

Die Haar- und Körperpflegemittel, wie beispielsweise Haarshampoos, Haarlotionen oder Dusch- und Schaumbäder, können als weitere Hilfs- und Zusatzstoffe Emulgatoren, Überfettungsmittel, Verdickungsmittel, Kationpolymere, Siliconverbindungen, biogene Wirkstoffe, Filmbildner, Konservierungsmittel, Farb- und Duftstoffe enthalten.

Als **Emulgatoren** kommen sowohl bekannte W/O- als auch O/W- Emulgatoren wie beispielsweise gehärtetes und ethoxyliertes Ricinusöl, Polyglycerinfettsäureester oder Polyglycerinpolyricinoleate bzw. Polyglycerinpoly-12-hydroxystearate in Frage. Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationischen Cellulosederivate, kationischen Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor- und/oder alkylmodifizierte Siliconverbindungen. Unter **biogenen Wirkstoffen** sind beispielsweise Pflanzenextrakte und Vitaminkomplexe zu verstehen. Gebräuchliche **Filmbild-ner** sind beispielsweise Chitosan, mikrokristallines Chitosan, quaterniertes Chitosan, Poly-vinylpyrrolidon, Vinylpyrrolidon-Vinylacetat-Copolymerisate, Polymere der Acrylsäure-reihe, quaternäre Cellulose-Derivate, Kollagen, Hyaluronsäure bzw. deren Salze und ähnliche Verbindungen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-% bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.

### Beispiele

### I. Herstellbeispiele

### Beispiel 1

In einem 2-l-Dreihalskolben mit Rührer, Rückflußkühler und Tropftrichter wurden bei 45°C 379 g (1 mol) technisches Sorbitanmonolaurat (Dehymuls® SML, Henkel KGaA, Düsseldorf/FRG), 330 g Wasser und 63 g Isopropylalkohol vorgelegt und durch Zugabe von 25 Gew.-%iger Natriumhydroxidlösung auf einen pH-Wert von 8 eingestellt. Anschließend wurde die Mischung auf 70°C erhitzt und innerhalb von 2 h mit 216,5 g (1 mol) Epoxypropyltrimethylammoniumchlorid (70 Gew-%ig in Wasser) versetzt. Die Mischung wurde weitere 48 h bei einem pH-Wert im Bereich von 7,5 bis 8,5 gerührt. Das Sorbitanesterquat wurde als flüssige, gelblich gefärbte Paste mit einem Feststoffgehalt von 52 Gew.-% und einem kationischen Aktivsubstanzanteil von 0,065 meq/g erhalten.

### Beispiel 2

Beispiel 1 wurde wiederholt, anstelle des Sorbitanmonolaurats jedoch 561 g (2 mol) technisches Sorbitandilaurat (Dehymuls® SDL, Henkel KGaA) eingesetzt. Das Sorbitandiesterquat wurde ebenfalls als flüssige, leicht gelblich gefärbte Paste erhalten.

### Beispiel 3

Beispiel 2 wurde wiederholt, jedoch 132 g (2 mol) Epoxypropyltrimethylammoniumchlorid eingesetzt. Das Sorbitandiesterdiquat wurde als viskose, gelblich gefärbte Paste erhalten.

### Beispiel 4

Beispiel 1 wurde wiederholt, anstelle des Sorbitanesters jedoch ein Polysorbat, nämlich 629 g (0,5 mol) eines Anlagerungsproduktes von durchschnittlich 20 Mol Ethylenoxid an ein technisches Sorbitanmonolaurat (Eumulgin® SML 20, Henkel KGaA) mit 75 g (0,5 mol) Epoxypropyltrimethylammoniumchlorid umgesetzt. Nach Verdünnen mit Wasser wurde eine flüssige, leicht gelb gefärbte Paste mit einem Feststoffgehalt von 60 Gew.-% erhalten.

### II. Anwendungsbeispiele Haarkosmetik

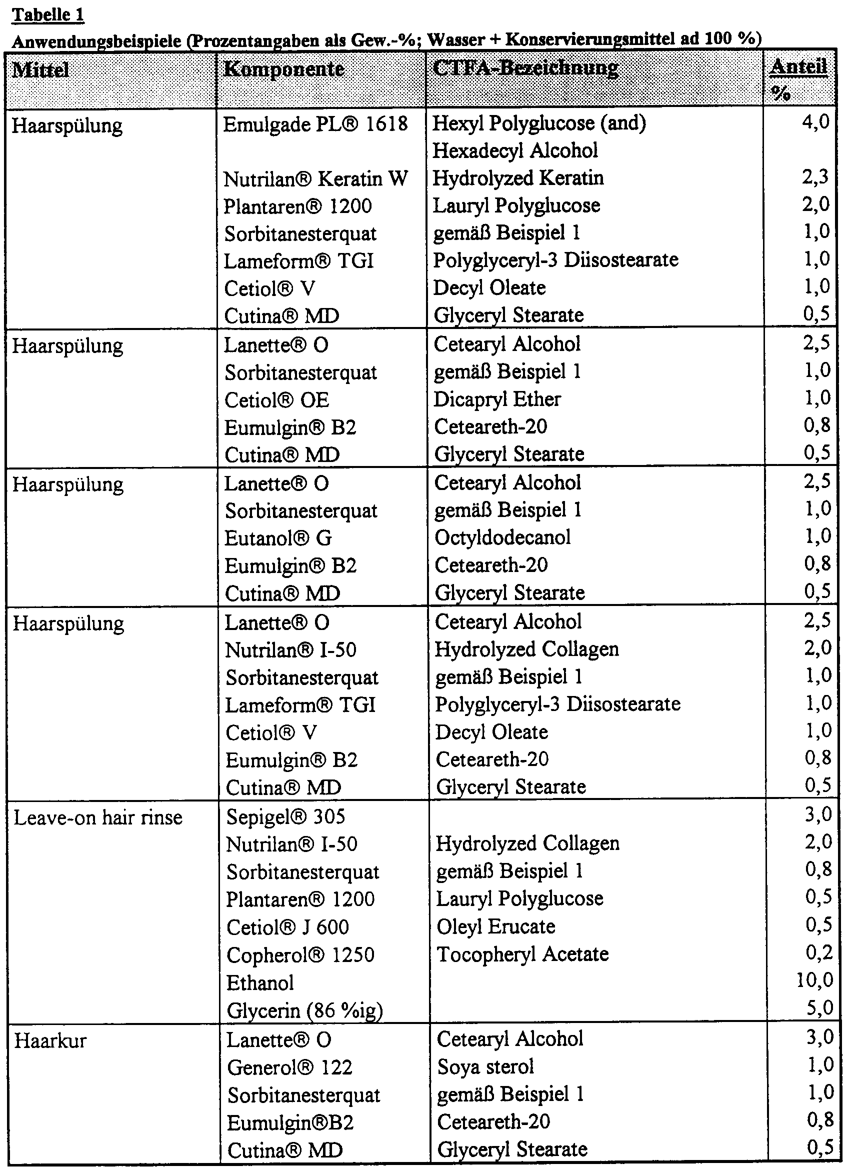

**Tabelle 1B**

| **Anwendungsbeispiele Haarkosmetik (Forts.)** | | | |
|---|---|---|---|
| **Mittel** | **Komponente** | **CTFA-Bezeichnung** | **Anteil** **%** |
| Haarkur | Lanette® O | Cetearyl Alcohol | 2,5 |
| | Sorbitanesterquat | gemäß Beispiel 1 | 1,5 |
| | Eumulgin® B2 | Ceteareth-20 | 1,0 |
| | Generol® 122 | Soya sterol | 1,0 |
| | Eutanol® G | Octyldodecanol | 1,0 |
| | Cutina® MD | Glyceryl Stearate | 0,5 |
| Duschbad | Texapon® K 14 S | Sodium Myreth Sulfate | 38,0 |
| | Plantaren® 2000 | Decyl Polyglucose | 7,0 |
| | Lamesoft® LMG | Glyceryl Laurate (and) Potassium Cocoyl Hydrol. Collagen | 3,0 |
| | Arylpon® F | Laureth-2 | 3,0 |
| | Sorbitanesterquat | gemäß Beispiel 1 | 0,5 |
| Duschbad | Texapon® NSO | Sodium Laureth Sulfate | 38,0 |
| | Plantaren® 2000 | Decyl Polyglucose | 7,0 |
| | Euperlan® PK 3000 | Glycol Distearate (and) Cocoamidopropyl Betaine | 3,0 |
| | Arlypon® F | Laureth-2 | 3,0 |
| | Lamesoft® LMG | Glyceryl Laurate (and) Potassium Cocoyl Hydrol. Collagen | 2,0 |
| | Sorbitanesterquat | gemäß Beispiel 1 | 0,5 |
| | Kochsalz | | 1,5 |
| Duschgel | Texapon® NSO | Sodium Laureth Sulfate | 25,0 |
| | Texapon® SB3 | Disodium Laurethsulfosuccinate | 10,0 |
| | Dehyton® K | Cocoamidopropyl Betaine | 10,0 |
| | Plantaren® 2000 | Decyl Polyglucose | 6,0 |
| | Euperlan® PK 3000 | Glycol Distearate (and) Cocoamidopropyl Betaine | 5,0 |
| | Lamesoft® LMG | Glycol Distearate (and) Cocoamidopropyl Betaine | 4,0 |
| | Cetiol® HE | PEG-7 Glyceryl Cocoate | 1,0 |
| | Arlypon® F | Laureth-2 | 1,0 |
| | Sorbitanesterquat | gemäß Beispiel 1 | 0,5 |
| Waschlotion | Plantaren® PS 10 | Sodium Laureth Sulfate (and) Lauryl Polyglucose | 16,0 |
| | Euperlan® PK 3000 | Glycol Distearate (and) Cocoamidopropyl Betaine | 5,0 |
| | Sorbitanesterquat | gemäß Beispiel 1 | 0,5 |
| | Kochsalz | | 1,5 |

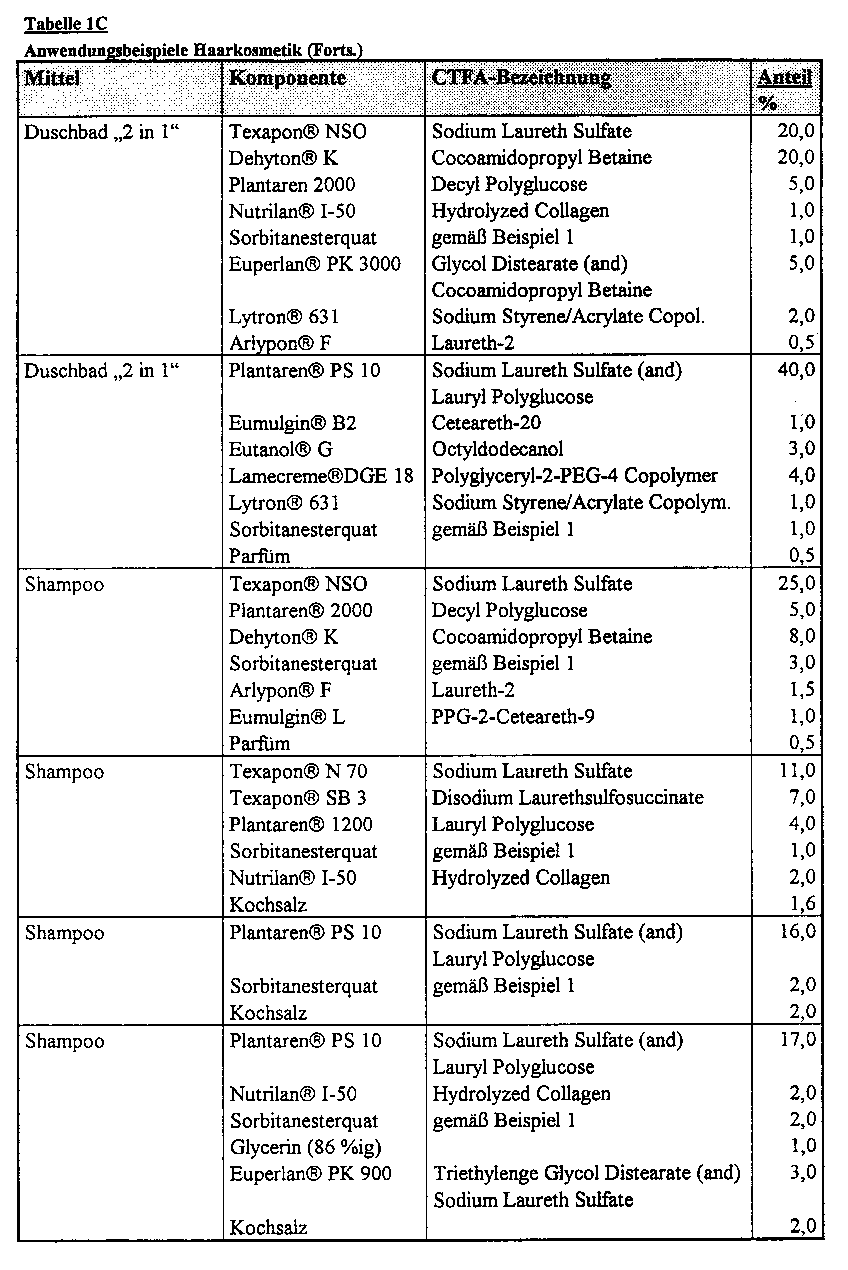

**Tabelle 1D**

| **Anwendungsbeispiele Haarkosmetik (Forts.)** | | | |
|---|---|---|---|
| **Mittel** | **Komponente** | **CTFA-Bezeichnung** | **Anteil** **%** |
| Shampoo | Texapon® ALS | Ammonium Laureth Sulfate | 23,0 |
| | Plantaren® 2000 | Decyl Polyglucose | 4,0 |
| | Dehyton® K | Cocoamidopropyl Betaine | 7,0 |
| | Sorbitanesterquat | gemäß Beispiel 1 | 2,0 |
| | Lamesoft® 156 | Hydrogenated Tallow Glycerides | 5,0 |
| | Monomuls® 90-L 12 | Glyceryl Laurate | 1,0 |
| | Kochsalz | | 3,0 |
| Schaumbad | Plantaren® PS 10 | Sodium Laureth Sulfate (and) Lauryl Polyglucose | 22,0 |
| | Dehyton® K | Cocoamidopropyl Betaine | 15,0 |
| | Sorbitanesterquat | gemäß Beispiel 1 | 3,0 |
| | Cetiol® HE | PEG-7 Glyceryl Cocoate | 2,0 |
| | Euperlan® PK 3000 | Glycol Distearate (and) Cocoamidopropyl Betaine | 5,0 |
| Schaumbad | Texapon® NSO | Sodium Laureth Sulfate | 30,0 |
| | Dehyton® K | Cocoamidopropyl Betaine | 10,0 |
| | Plantaren® 1200 | Lauryl Polyglucose | 10,0 |
| | Lamesoft® LMG | Glyceryl Laurate (and) Potassium Cocoyl Hydrol. Collagen | 4,0 |
| | Sorbitanesterquat | gemäß Beispiel 1 | 2,0 |
| | Guadin® AGP | Hydrolyzed Wheat Protein | 0,5 |
| Schaumbad | Melissenöl | | 5,0 |
| | Eumulgin® L | PPG-2-Ceteareth-9 | 15,0 |
| | Plantaren® 2000 | Decyl Polyglucose | 30,0 |
| | Dehyton® K | Cocoamidopropyl Betaine | 10,0 |
| | Sorbitanesterquat | gemäß Beispiel 1 | 4,0 |
| | Propylenglycol | Laureth-2 | 4,0 |
| | Arlypon® F | | 1,5 |

## Patentansprüche

1. Sorbitanesterquats, dadurch erhältlich, daß man Sorbitanester, gegebenenfalls nach Ethoxylierung, mit Epoxypropylammoniumverbindungen quaterniert.

2. Verfahren zur Herstellung von Sorbitanesterquats, bei dem man Sorbitanester, gegebenenfalls nach Ethoxylierung, mit Epoxypropylammoniumverbindungen quater-niert.

3. Verfahren nach Anspruch 2, **dadurch gekennzeichnet**, daß man Sorbitanester der Formel **(I)** einsetzt, in der 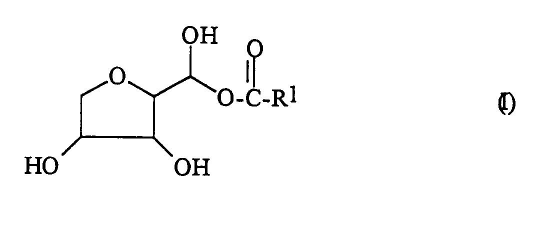 in der R¹CO für einen aliphatischen Acylrest mit 6 bis 22 Kohlenstoffatomen und 0 und/oder 1, 2 oder 3 Doppelbindungen steht.

4. Verfahren nach den Ansprüchen 2 und 3, **dadurch gekennzeichnet**, daß man Epoxypropylammoniumverbindungen der Formel **(II)** einsetzt, 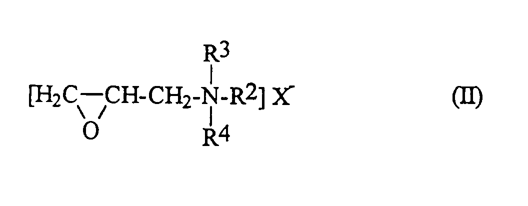 in der R², R³ und R⁴ unabhängig voneinander für gegebenenfalls hydroxysubstituierte Alkylreste mit 1 bis 4 Kohlenstoffatomen und X für Halogen steht.

5. Verwendung von Sorbitanesterquats nach Anspruch 1 zur Herstellung von oberflächenaktiven Mitteln.
